# EUROPÄISCHE PATENTSCHRIFT

(11) **EP 2 092 298 B1**
(45) Veröffentlichungstag und Bekanntmachung des Hinweises auf die Patenterteilung: **03.06.2015**
(21) Anmeldenummer: 07817767.2
(22) Anmeldetag: 02.11.2007
(51) Int. Cl.: G01N 21/03, G01N 21/05, G01N 21/35, A61B 5/097, B29C 45/16, B29L 31/00

(54) **VERFAHREN ZUR HERSTELLUNG EINER MESSGASKÜVETTE UND MIT DIESEM VERFAHREN HERGESTELLTE MESSGASKÜVETTE**
METHOD FOR MANUFACTURING A MEASUREMENT GAS CELL AND MEASUREMENT GAS CELL PRODUCED BY SAID METHOD
PROCÉDÉ DE FABRICATION D'UNE CUVETTE DE MESURE DE GAZ ET CUVETTE FABRIQUÉE PAR LEDIT PROCÉDÉ

(30) Priorität: 10.11.2006 DE 102006052999
(43) Veröffentlichungstag der Anmeldung: 26.08.2009
(73) Patentinhaber: Dräger Medical GmbH, 23558 Lübeck (DE)
(72) Erfinder: TAPPEHORN, Ludger, 23560 Lübeck (DE); VAN ZYL, Petrus Stephanus, 50374 Erftstadt (DE); BRANDT, Christiane, 23554 Lübeck (DE); LÜNSE, Jennifer, 23568 Lübeck (DE); HATTENDORFF, Horst-Dieter, 23611 Bad Schwartau (DE)
(86) Internationale Anmeldenummer: PCT/DE2007/001976
(87) Internationale Veröffentlichungsnummer: WO 2008/055478

(56) Entgegenhaltungen:
- WO-A-2004/081612
- WO-A-2004/096043
- US-A- 4 011 859

## Beschreibung

Die Erfindung betrifft ein Verfahren zur Herstellung einer Meßgasküvette für die Konzentrationsmessung von Gasbestandteilen mit den Merkmalen von Anspruch 1 und eine durch dieses Verfahren hergestellte Messgaskrüvette gemäß Anspruch 4.

Messeinrichtungen für eine Konzentrationsmessung von Gasbestandteilen des Atemstromes eines Patienten sind aus der klinischen Praxis bekannt. Hier gelangen sie insbesondere bei dem medizinischen Verfahren der Kapnometrie zum Einsatz, bei dem der Kohlenstoffdioxidgehalt in der Ausatemluft eines Patienten gemessen und überwacht wird. Anhand der Kohlendioxidkonzentration der Ausatemluft lässt sich die Beatmung relativ gut an den Patienten anpassen. Auch ist frühzeitig zu erkennen, ob ein Endotrachealtubus richtig liegt und ob die Stoffwechsellage des Patienten stimmt. Der letzte Punkt ermöglicht frühzeitiges Eingreifen bei bestimmten Komplikationen, wie einer malignen Hyperthermie oder gibt einen Anhaltswert für die Effektivität einer Reanimation.

Für die Konzentrationsmessung von Gasbestandteilen des Atemstromes wird vorzugsweise das Hauptstromverfahren angewendet, bei dem eine Meßgasküvette in einen Beatmungsstrom zwischen dem Respirationstrakt eines Patienten und einer Atemvorrichtung vorgesehen wird. Dazu wird ein den Atemvolumenstrom durchstrahlendes Infrarotmessverfahren verwendet, wie es beispielsweise in der DE 19 520 488 C1 offenbart ist, bei dem mittels geeigneter Infrarotstrahlungsdetektoren die konzentrationsabhängige Absorption der von einer Infrarotstrahlungsquelle ausgesendeten Infrarotstrahlung nach Durchquerung des Atemstromes in der Meßgasküvette für eine, für ein bestimmtes Gas wie CO2, charakteristische Wellenlänge erfasst wird. Aus dem empfangenen Messsignal lässt sich die Konzentration des bestimmten Gases rechnerisch ermitteln. Die Meßgasküvette weißt dazu zwei senkrecht zur Strömungsrichtung angeordnete, gegenüberliegende Fenster auf, durch die infrarotes Licht gesendet wird.

Aus der WO2004/096043 A1 ist eine Meßgasküvette für eine Konzentrationsmessung von Gasbestandteilen der Atemluft bekannt, bei der ein Meßgasküvettengehäuse und Fenster, durch die ein infrarotes Licht gesendet wird, separat gefertigt sind. Die Fenster werden mit einem Spritzgussverfahren hergestellt und im Anschluss mit dem Gehäuse durch ein geeignetes Klebeverfahren verbunden. Für die Berechnung der Konzentration eines bestimmten Gases ist ein definierter Abstand der Fenster zueinander essentiell. Der Abstand definiert ein inneres Volumen der Meßgasküvette, welches in die Berechnung der Konzentration der Gasbestandteile der Atemluft einfließt. Durch das Einkleben der Fenster kann es zu Abweichungen des Fensterabstandes von einem definierten Wert kommen und damit zu Ungenauigkeiten der berechneten Konzentrationswerte.

Des Weiteren weisen die Fenster eine sehr geringe Wandstärke auf. Eine geringe Wandstärke der Fenster ist zwar für deren Transmissionsverhalten bezüglich des infraroten Lichtes vorteilhaft, aber es kann infolge eines plötzlichen Druckwechsels im Respirationskreislauf aufgrund der geringen Wandstärke zu einer Bewegung der Fenster kommen, die eine Messwertverfälschung zur Folge haben kann, da dabei der Abstand der Fenster zueinander variiert. Die Fenster sind mit einer nach außen gewölbten Seite ausgeführt, die in Richtung des Inneren der Meßgasküvette an dem Gehäuse befestigt sind. Für die Herstellung der Meßgasküvette ist eine hochpräzise Fertigung erforderlich, um Unebenheiten infolge eines Überstehens der Fenster im Bereich eines von den Fenstern gebildeten, mit Atemluft des Patienten durchströmenden Kanals, im Inneren der Meßgasküvette zu vermeiden.

Weiterhin wird an den Kleber eine hohe Anforderung hinsichtlich Biokompatibilität gestellt.

Die Aufgabe der Erfindung besteht demnach in der Bereitstellung eines einfachen Verfahrens zur Herstellung einer Meßgasküvette und einer mit diesem Verfahren einfach herzustellenden Meßgaskürvette.

Diese Aufgabe wird gelöst durch eine gemäß dem erfindungsgemäßen Verfahren hergestellte Meßgasküvette. bestehend aus einem dünnwandig ausgebildeten inneren Teil, welches zwei gegenüberliegende, den Atemstrom begrenzende Wände aufweist, wobei die Wände für infrarotes Licht durchlässig sind und einem äußeren Teil, welches in ein Mittelstück, in ein Eingangs- und ein Ausgangsstück unterteilt ist. Das Eingangsstück ist als Verbinder zur Verbindung mit einem zu einem Patienten führenden Atemschlauch und das Ausgangsstück als Verbinder zur Verbindung mit einem zu einer Atemvorrichtung führenden Atemschlauch ausgeführt. Das Mittelstück weist zwei gegenüberliegende Aussparungen auf, die mit den Wänden des inneren Teils jeweils ein Fenster bilden, wobei der Bereich außerhalb der Fenster des inneren Teils thermisch mit dem äußeren Teil verbunden ist und der äußere Teil den inneren Teil vollständig umschließt.

Das erfindungsgemäße Verfahren zur Herstellung der Meßgasküvette weist folgende Verfahrensschritte auf: a) Herstellen eines dünnwandigen Teils mit zwei gegenüberliegenden Wänden in einer ersten Stufe eines Spritzgussverfahrens, welches einen inneren Teil der Meßgasküvette bildet, b) Einbringen von einem Platzhalter für eine Freihaltung von Spritzgussmaterial an einem ersten und einem zweiten von Spritzgussmaterial freizuhaltenden Platz, an den gegenüberliegenden Wänden des inneren Teils, wobei der Platzhalter so ausgerichtet ist, dass der erste von Material freizuhaltende Platz mit dem zweiten von Material freizuhaltenden Platz in einer Flucht liegt und c) Herstellen eines äußeren Teils der Meßgasküvette in einer zweiten Stufe des Spritzgussverfahrens, in dem zumindest das innere Teil (11) vollständig mit Material umspritzt wird so dass eine thermische Verbindung der beiden Teile erfolgt, wobei an den Stellen der Platzhalter zwei gegenüberliegende, den Atemstrom begrenzende Fenster ausgebildet werden.

Vorteilhafte Ausführungen und Weiterbildungen der Erfindung sind in den Unteransprüchen angegeben.

Die mit der Erfindung erzielten Vorteile bestehen insbesondere darin, dass durch das erfindungsgemäße Verfahren ein sehr kostengünstiges Herstellverfahren zur Herstellung der Meßgasküvetten ermöglicht wird. Ein zusätzlicher Arbeitsgang zum Einbringen der Fenster in die Meßgasküvetten entfällt.

In einer bevorzugten Ausführungsform besteht das innere Teil vorzugsweise aus einem Random Copolymer, wodurch eine hohe Transmission des infraroten Lichtes, speziell in einem Wellenlängenbereich von 4 bis 5 Mikrometer, ermöglicht wird.

Mit dem erfindungsgemäßen Verfahren zur Herstellung der Meßgasküvette lassen sich zudem Wandstärken des inneren Teils im Bereich von 170 bis 210 Mikrometer realisieren, so dass die durch die Verbindung des inneren Teils mit dem äußeren Teil gebildeten Fenster, bei Druckwechsel im Respirationskreislauf, eine gute Stabilität aufweisen und Bewegungen sowie Deformationen der Fenster weitestgehend vermieden werden können.

Das innere Teil der Meßgasküvette ist vorzugsweise U-förmig ausgebildet, derart, dass die gegenüberliegenden Wände über einen Steg miteinander verbunden sind. Es kann aber auch in einer weiteren Ausführung eine hülsenförmige Ausbildung des inneren Teils vorgesehen sein. Vorzugsweise erstreckt sich das innere Teil über die eigentliche Meßgasküvette bis in das Eingangs- und / oder das Ausgangsstück des äußeren Teils. Damit wird in vorteilhafter Weise eine glatte Oberfläche des mit Atemluft des Patienten durchströmten Kanals gebildet und Turbulenzen infolge von Unebenheiten in dem Kanal ausgeschlossen.

Ausführungsbeispiele der Erfindung sind in den Figuren 1 bis 5 beschrieben. Dabei zeigen:
- Fig. 1: eine Messanordnung eines Hauptstromverfahrens für eine Konzentrationsmessung von Gasbestandteilen des Atemstromes eines Patienten,
- Fig. 2: eine Seitenansicht in Längsrichtung der erfindungsgemäßen Meßgasküvette,
- Fig. 3: eine Schnittdarstellung der erfindungsgemäßen Meßgasküvette,
- Fig. 4: eine Einzelheit "A" von Fig. 3 schematisch und in größerem Maßstab und
- Fig. 5: einen Querschnitt durch den Mittelteil der erfindungsgemäßen Meßgasküvette in einer Ausführungsform.

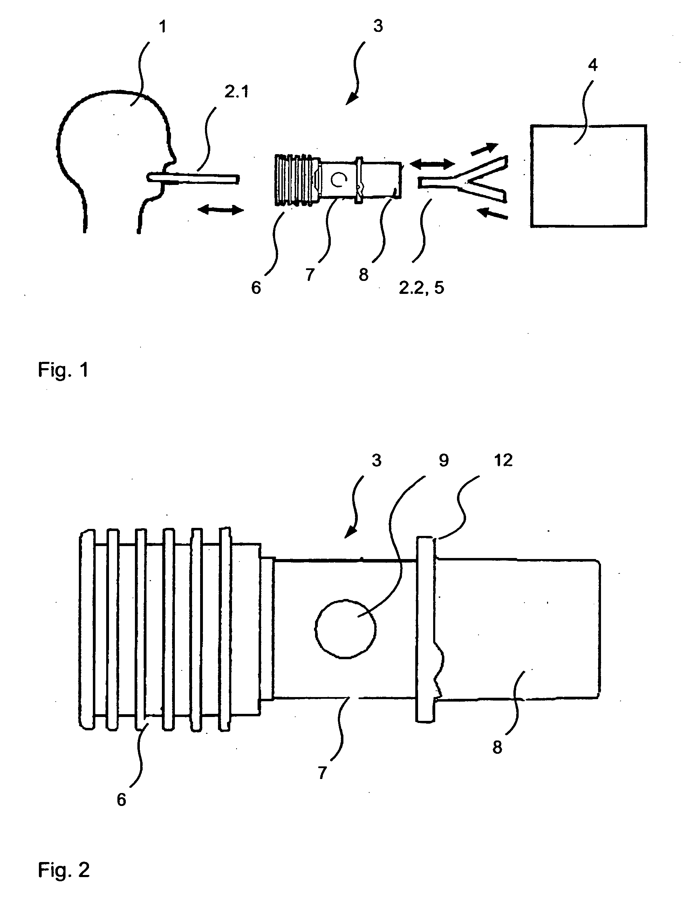

In Fig. 1 ist eine Messanordnung eines Hauptstromverfahrens für eine Konzentrationsmessung von Gasbestandteilen eines Atemstromes eines Patienten 1, beispielsweise von CO₂, dargestellt. Zwischen einer den Patenten 1 mit Atemgas versorgenden Atemvorrichtung 4 ist eine Meßgasküvette 3 geschaltet, durch die die von dem Patienten 1 ein- und ausgeatmete Luft strömt. Die Atemvorrichtung 4 kann ein Anästhesiegerät oder ein Beatmungsgerät umfassen. Für eine Messung wird vorzugsweise die Infrarot-Spektroskopie angewendet. Ein entsprechendes Messgerät ist an der Meßgasküvette 3 vorgesehen und nicht explizit dargestellt. Auf diese Weise kann die CO₂ Konzentration des von und zu dem Patienten 1 strömenden Atemgases kontinuierlich ausgewertet werden.

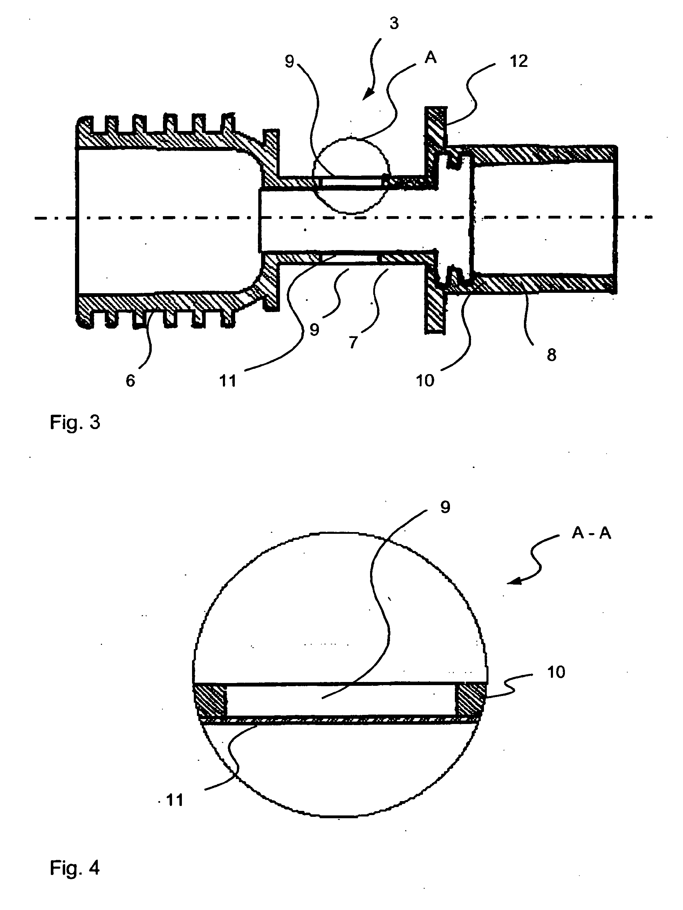

In Fig. 2 ist eine Seitenansicht einer erfindungsgemäßen Meßgasküvette 3 dargestellt. Die Meßgasküvette 3 umfasst ein Eingangsstück 6, ein Mittelstück 7 und ein Ausgangstück 8. Das Eingangsstück 6 ist vorzugsweise mit einem zu dem Patienten 1 führenden Atemschlauch 2.1 verbunden (Fig. 1), wohingegen das Ausgangsstück 8 mit der Atemvorrichtung 4 über den Atemschlauch 2.2 gekoppelt ist. Zwischen Ausgangsstück 8 und Atemvorrichtung 4 kann dabei noch ein so genanntes Y-Stück 5 vorgesehen sein, welches die Atemluft in einen inspiratorischen Teil und einen exspiratorischen Teil unterteilt. Das Eingangsstück 6 ist konisch gestaltet und weist in Umfangsrichtung parallel verlaufende Rillen auf. Dadurch kann der patientenseitige Atemschlauch 2.1 sicher mit dem Eingangsstück 6 der Meßgasküvette 3 verbunden werden. Zudem wird aufgrund der Formgestaltung des Eingangsstücks 6 weniger Material für dessen Herstellung benötigt. Das Ausgangsstück 8 ist für eine sichere Verbindung mit dem geräteseitigen Atemschlauch 2.2 bzw. mit dem Y-Stück 5 ebenfalls konisch ausgeführt. Vorzugsweise ist zur Vermeidung einer Verwechslung der Anschlüsse der Meßgasküvette 3 in einer bevorzugten Ausführung der Durchmesser des Eingangsstückes 6 größer als der Durchmesser des Ausgangsstückes 8 vorgesehen. Die Meßgasküvette 3 weist im Bereich des Mittelstückes 7 zwei gegenüberliegende Fenster 9 auf, durch die infrarotes Licht gesendet wird. Ein Aufbau der Fenster 9 ist in den Schnittdarstellungen der Figuren 3 und 4 gezeigt. Die Fenster 9 werden von dem dünnwandig ausgebildeten inneren Teil 11 und dem das innere Teil 11 umgebenden äußeren Teil 10, durch zwei gegenüberliegende, den Atemstrom begrenzende Wände 16, des inneren Teils 11 und zwei gegenüberliegende Aussparungen des Mittelstücks 7 des äußeren Teils 10 gebildet. Für die Infrarot-Spektroskopie Messung wird die nicht gezeigte Messeinrichtung so an der Meßgasküvette 3 an einem Befestigungsmittel 12 positioniert, dass ein in einem IR-Sender erzeugtes infrarotes Licht die Meßgasküvette 3 durch die beiden Fenster 9 passiert. In einer gegenüberliegend angeordneten, nicht näher dargestellten IR-Detektoreinheit, bestehend aus einem IR-Messdetektor mit CO₂ Filter, einem Referenzdetektor mit entsprechendem Filter und einem Strahlenteiler wird das infrarote Licht empfangen und in einer nicht näher dargestellten Auswerteeinheit, ausgewertet. Eine Auswertung erfolgt auf der Grundlage des Absorptionsverhaltens des infraroten Lichtes entsprechend der Konzentration des CO₂-Gases in einem für CO₂ spezifischen Spektralbereich. Eine Messung findet speziell bei einer Wellenlänge von ca. 4,25 bis 4,3 Mikrometer statt. Die Referenzmessung mittels des Referenzdetektors erfolgt mit einer Wellenlänge, bei der CO₂ keine Absorption aufweist. Aus den gemessenen Werten wird der Konzentrationswert von CO₂ im Atemgas bestimmt.

Die Meßgasküvette 3 ist in der in den Figuren 3 und 4 dargestellten Ausführung so gestaltet, dass das innere Teil 11 sich bis in das Eingangsstück 6 und / oder das Ausgangsstück 8 des äußeren Teils 10 erstreckt. Damit werden mit dem dünnwandig ausgebildeten inneren Teil 11 eine glatte Oberfläche eines mit Atemluft des Patienten durchströmenden Kanals der Meßgasküvette 3 erzeugt und Turbulenzen der Atemluft infolge von Hindernissen in dem Kanal vermieden. Die durch das innere und äußere Teil 11, 10 gebildeten Fenster 9 sind, einerseits aufgrund der Materialstärke des inneren Teils 11 von vorzugsweise 180 Mikrometer sehr dünn ausgebildet und bieten daher eine hohe Transmission des infraroten Lichtes, andererseits wird eine ausreichend gute Stabilität und Steifigkeit durch das das innere Teil 11 umgebende äußere Teil 10 erzeugt, so dass es nicht zu Verformungen bei ändernden Atemluftverhältnissen und damit zu fehlerbehafteten Messwerten kommen kann. Der Atemluftkanal der Meßgasküvette 3 ist im mittleren Teil vorzugsweise quaderförmig ausgebildet, wohingegen in dem Eingangsstück 6 und dem Ausgangsstück 8 eine zylindrische Ausbildung vorgesehen ist.

Das Messverfahren der Hauptstrommessung ist mit einer Vergrößerung des nicht am Gasaustausch beteiligten Totraumes verbunden. Zur Kompensation dieses Totraumes muss ein höheres Volumen pro Atemzug appliziert werden, d.h. ein Beatmungsdruck muss erhöht werden. Bei Neugeborenen kann ein erhöhter Wert des Beatmungsdrucks über einen größeren Zeitabstand jedoch zu einer Verletzung der noch nicht vollständig ausgebildeten Lungen führen. Eine weitere Ausgestaltung der erfindungsgemäßen Meßgasküvette 3 sieht daher einen Atemluftkanal mit geringerem Querschnitt vor, um den nicht am Gasaustausch beteiligten Totraum zu reduzieren und eine Verletzung der Lunge, speziell bei Neugeborenen und Kindern, zu vermeiden.

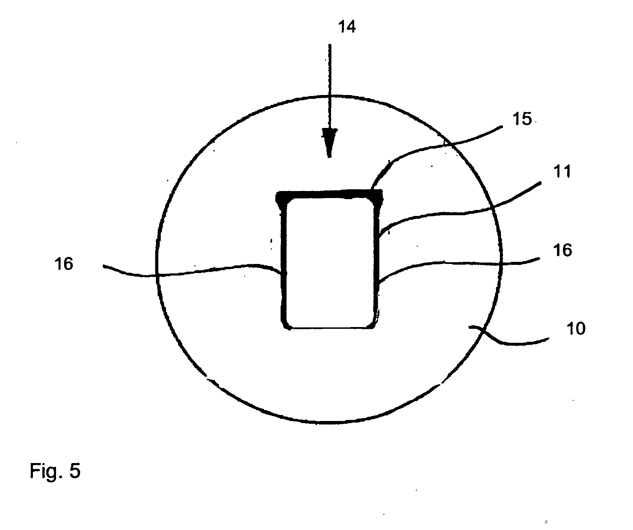

Das erfindungsgemäße Verfahren zur Herstellung der Meßgasküvette 3 wird im Folgenden anhand des in Fig. 5 dargestellten Ausführungsbeispiels erläutert. In Fig. 5 ist ein Querschnitt durch das Mittelstück 7 einer Meßgasküvette 3 gezeigt, bei der das innere Teil 11 U-förmig ausgebildet ist, in der Art, dass die gegenüberliegenden Wände 16 über einen Steg 15 miteinander verbunden sind und das äußere Teil 10 das innere Teil 11 umgibt und zwei senkrecht zur Strömungsrichtung angeordnete, gegenüberliegende Aussparungen aufweist, die mit den über den Steg 15 verbundenen gegenüberliegenden Wänden 16 des inneren Teils 11 jeweils ein Fenster 9 bilden. Die erfindungsgemäße Meßgasküvette 3 wird mittels Spritzgussverfahren hergestellt. In einem ersten Verfahrensschritt wird ein dünnwandiges Teil 11 mit zwei gegenüberliegenden Wänden 16 in einem Spritzgusswerkzeug erzeugt, welches das innere Teil 11 der Meßgasküvette 3 bildet. Ein Anspritzpunkt wird mittig, oberhalb eines Stegs 15 vorgesehen. Hierdurch wird ein Materialstau in dem stegseitigen Bereich des inneren Teils 11 vermieden und das Material kann gleichmäßig über die Bereiche der zu erzeugenden Wänden 16 des herzustellenden inneren Teils 11 fließen. Dadurch lässt sich eine homogene Struktur der gegenüberliegenden Wände 16 des inneren Teils 11 erzielen. Die Homogenität der Wände 16 verhindert systembedingte Messfehler aufgrund unterschiedlichen Transmissionsverhaltens des Wandmaterials der Wände 16. Das Spritzgussmaterial muss somit nicht eine ganze Spritzgussform, welche zur Erzeugung des äußeren Teils 10 vorgesehen wird, durchqueren, sondern verteilt sich nur in einem Bereich, der für eine Herstellung der gegenüberliegenden Wände 16 des inneren Teils 11 erforderlich ist. Dadurch lassen sich signifikant geringere Wandstärken erreichen. Ein zusätzliches Temperieren des Spitzgusswerkzeuges verhindert zudem ein vorzeitiges Auskühlen in den Bereichen, in denen das Spritzgusswerkzeug bereits vollständig mit Spritzgussmaterial ausgefüllt ist.

In einem weiteren Verfahrensschritt wird jeweils ein Platzhalter für eine Freihaltung von Spritzgussmaterial an den gegenüberliegenden Wänden 16 des inneren Teils 11 vorgesehen und so ausgerichtet, dass der von Material freizuhaltende Platz in einer Flucht liegt. Damit werden zwei Bereiche an den gegenüberliegenden Wänden 16 des inneren Teils 11 von Spritzgussmaterial freigehalten und in einem letzten Verfahrensschritt und einer zweiten Stufe des Spritzgussverfahrens wird zumindest das innere Teil 11 vollständig mit Material umspritzt, so dass zwei senkrecht zur Strömungsrichtung angeordnete, gegenüberliegende, den Atemstrom begrenzende Fenster 9 an den Stellen der Platzhalter ausgebildet werden. Dabei verbindet sich das Material des inneren Teils 11 mit dem aufgespritzten Material, das den äußeren Teil 10 der Meßgasküvette 3 bildet.

Die Herstellung der Meßgasküvette 3 erfolgt somit in einer Spritzgussvorrichtung einteilig. Mit dem Verfahren zur Herstellung der erfindungsgemäßen Meßgasküvette 3 wird zuerst das innere Teil 11 in einem Stück gespritzt und im Anschluss der äußere Körper 10 der Meßgasküvette 3 in Form des äußeren Teils 10 um das innere Teil 11 gespritzt und somit thermisch mit dem inneren Teil 11 verbunden.

### BEZUGSZEICHENLISTE

- 1: Patient
- 2.1: Atemschlauch zu einem Patienten
- 2.2: Atemschlauch zu einer Atemvorrichtung
- 3: Meßgasküvette
- 4: Atemvorrichtung
- 5: Y-Stück
- 6: Eingangsstück
- 7: Mittelstück
- 8: Ausgangsstück
- 9: Fenster
- 10: Äußerer Teil
- 11: Innerer Teil
- 12: Befestigungsmittel
- 14: Fliessrichtung des Spritzgussmaterials
- 15: Steg
- 16: Wände des inneren Teils

## Patentansprüche

1. Verfahren zur Herstellung einer Messgasküvette (3) für eine Messung der Konzentration von Gasbestandteilen des Atemstroms eines Patienten (1), **gekennzeichnet durch** die folgenden Verfahrensschritte:
a) Herstellen eines dünnwandigen Teils mit zwei gegenüberliegenden Wänden (16) in einer ersten Stufe eines Spritzgussverfahrens, welches einen inneren Teil (11) der Messgasküvette (3) bildet,
b) Einbringen von einem Platzhalter für eine Freihaltung von Spritzgussmaterial an einem ersten und einem zweiten von Spritzgussmaterial freizuhaltenden Bereich der gegenüberliegenden Wände (16) des inneren Teils (11), wobei der Platzhalter so ausgerichtet ist, dass der erste von Material freizuhaltende Bereich mit dem zweiten von Material freizuhaltenden Bereich in einer Flucht liegt und
c) Herstellen eines äußeren Teils (10) der Messgasküvette (3) in einer zweiten Stufe des Spritzgussverfahrens, in dem das innere Teil (11) vollständig mit Material umspritzt wird, wobei an den Stellen der Platzhalter zwei gegenüberliegende, den Atemstrom begrenzende Fenster (9) ausgebildet werden.

2. Verfahren nach Anspruch 1, **dadurch gekennzeichnet, dass** das Spritzgusswerkzeug derart gestaltet ist, dass im Verfahrensschritt a) ein Einspritzpunkt zum Herstellen des inneren Teils (11) oberhalb eines Steges (15) vorgesehen ist, wobei der Steg (15) die zwei gegenüberliegenden Wänden (16) verbindet.

3. Verfahren nach Anspruch 2, **dadurch gekennzeichnet, dass** der Einspritzpunkt zum Herstellen des inneren Teils (11) mittig bezogen auf den Steg (15) angeordnet ist.

4. Messgasküvette (3) für die Messung der Konzentration von Gasbestandteilen eines Atemstroms eines Patienten (1) bestehend aus
einem inneren Teil (11) mit zwei gegenüberliegenden, den Atemstrom begrenzenden Wänden (16), die für infrarotes Licht durchlässig sind, und
einem äußeren Teil (10) mit zwei gegenüberliegenden Aussparungen in seinem mittleren Bereich, die mit den Wänden (16) des inneren Teils (11) jeweils ein Fenster (9) bilden, wobei das äußere Teil (10) das innere Teil (11) umgibt,
**dadurch gekennzeichnet, dass**
die Messgasküvette (3) durch ein Verfahren gemäß Anspruch 1 hergestellt sind.

5. Messgasküvette nach Anspruch 4 , **dadurch gekennzeichnet, dass** das innere Teil (11) und das äußere Teil (10) aus dem gleichen Material ausgeführt sind.

6. Messgasküvette nach Anspruch 4 , **dadurch gekennzeichnet, dass** das innere Teil (11) und das äußere Teil (10) aus einem Material der Gruppe der Polyolefine ausgeführt sind, wobei das innere Teil (11) vorzugsweise aus einem Random-Copolymer und das äußere Teil (10) vorzugsweise aus einem Impact-Copolymer besteht.

7. Messgasküvette nach einem der Ansprüche 4 bis 6, **dadurch gekennzeichnet, dass** eine Wandstärke des inneren Teils (11) im Bereich von 170 bis 210 Mikrometer liegt und vorzugsweise 180 Mikrometer beträgt.

8. Messgasküvette nach einem der Ansprüche 4 bis 7, **dadurch gekennzeichnet, dass** das innere Teil (11) U-förmig ausgebildet ist derart, dass die gegenüberliegenden Wände (16) über einen Steg (15) miteinander verbunden sind.

9. Messgasküvette nach einem Ansprüche 4 bis 7, **dadurch gekennzeichnet, dass** das innere Teil (11) hülsenförmig ausgebildet ist.

10. Messgasküvette nach einem der Ansprüche 4 bis 9, **dadurch gekennzeichnet, dass** der mittlere Bereich (7) des äußeren Teils (10) Mittel (12) zur Befestigung einer Messvorrichtung aufweist.

11. Messgasküvette nach einem der Ansprüche 4 bis 10, **dadurch gekennzeichnet, dass** das äußere Teil (10) ein Eingangsstück (6) und ein Ausgangsstück (8) aufweist, wobei vorzugsweise an dem Eingangsstück (6) in Umfangsrichtung parallel verlaufende Rillen ausgebildet sind.

12. Messgasküvette nach Anspruch 11, **dadurch gekennzeichnet, dass** das Eingangsstück (6) und / oder das Ausgangsstück (8) konisch ausgeführt ist.

13. Messgasküvette nach einem der Ansprüche 11 oder 12, **dadurch gekennzeichnet, dass** sich das innere Teil (11) bis in das Eingangsstück (6) und / oder das Ausgangsstück (8) des äußeren Teils (10) erstreckt.

14. Verwendung einer Messgasküvette (3) nach einem der Ansprüche 4 bis 13 für eine Messung der Konzentration von Gasbestandteilen des Atemstroms eines Patienten (1), mit einer Messeinrichtung, die die Atemgase, welche zu und von einer Atemvorrichtung (4) zur Unterstützung der Beatmung des Patienten (1) strömen, bestimmt.

15. Verwendung einer Messgasküvette (3) nach Anspruch 14, **dadurch gekennzeichnet, dass** das Messverfahren auf einer Infrarot-Lichtabsorptionsmessung beruht.

## Claims

1. A method for producing a measurement gas cuvette (3) for measuring the concentration of gas constituents of the respiratory flow of a patient (1), **characterised by** the following method steps:
a) production of a thin-walled portion having two opposing walls (16) in a first stage of an injection-moulding process that forms an inner portion (11) of the measurement gas cuvette (3),
b) introduction of a place-holder for keeping injection-moulding material away from a first and a second region of the opposing walls (16) of the inner portion (11) to be kept free of injection-moulding material, wherein the place-holder is brought into line in such a way that the first region that is to be kept free of material is in alignment with the second region that is to be kept free of material, and
c) production of an outer portion (10) of the measurement gas cuvette (3) in a second stage of the injection-moulding process in which material is injection-moulded completely around the inner portion (11), wherein two opposing windows (9) that delimit the respiratory flow are formed at the sites of the place-holders.

2. A method according to claim 1, **characterised in that** the injection-moulding die is configured in such a way that in method step a) an injection point for producing the inner portion (11) is provided above a web (15), wherein the web (15) connects the two opposing walls (16).

3. A method according to claim 2, **characterised in that** the injection point for producing the inner portion (11) is arranged centrally in relation to the web (15).

4. A measurement gas cuvette (3) for measuring the concentration of gas constituents of a respiratory flow of a patient (1), consisting of
an inner portion (11) having two opposing walls (16) that delimit the respiratory flow and are permeable to infrared light, and
an outer portion (10) having two opposing recesses in its central region which with the walls (16) of the inner portion (11) in each case form a window (9), wherein the outer portion (10) surrounds the inner portion (11),
.**characterised in that**
the measurement gas cuvette (3) is produced by a method in accordance with claim 1.

5. A measurement gas cuvette according to claim 4, **characterised in that** the inner portion (11) and the outer portion (10) are constructed from the same material.

6. A measurement gas cuvette according to claim 4, **characterised in that** the inner portion (11) and the outer portion (10) are constructed from a material of the polyolefin group, wherein the inner portion (11) preferably consists of a random copolymer, and the outer portion (10) preferably consists of an impact copolymer.

7. A measurement gas cuvette according to one of claims 4 to 6, **characterised in that** a wall thickness of the inner portion (11) lies in the range of 170 to 210 micrometres and preferably amounts to 180 micrometres.

8. A measurement gas cuvette according to one of claims 4 to 7, **characterised in that** the inner portion (11) is formed in a U-shape in such a way that the opposing walls (16) are connected together by way of a web (15).

9. A measurement gas cuvette according to one of claims 4 to 7, **characterised in that** the inner portion (11) is formed in the shape of a sleeve.

10. A measurement gas cuvette according to one of claims 4 to 9, **characterised in that** the central region (7) of the outer portion (10) has means (12) for securing a measurement apparatus.

11. A measurement gas cuvette according to one of claims 4 to 10, **characterised in that** the outer portion (10) has an inlet piece (6) and an outlet piece (8), wherein grooves running in parallel in the peripheral direction are preferably formed on the inlet piece (6).

12. A measurement gas cuvette according to claim 11, **characterised in that** the inlet piece (6) and/or the outlet piece (8) are/is formed in a conical manner.

13. A measurement gas cuvette according to one of claims 11 or 12, **characterised in that** the inner portion (11) extends up into the inlet piece (6) and/or the outlet piece (8) of the outer portion (10).

14. Use of a measurement gas cuvette (3) according to one of claims 4 to 13 for measuring the concentration of gas constituents of the respiratory flow of a patient (1), with a measurement device that determines the respiratory gases which flow to and from a respiratory apparatus (4) to support the respiration of the patient (1).

15. Use of a measurement gas cuvette (3) according to claim 14, **characterised in that** the measurement method is based on infrared-light absorption measurement.

## Revendications

1. Procédé de fabrication d'une cuvette de gaz à mesurer (3) destinée à mesurer la concentration de composants gazeux du flux respiratoire d'un patient (1), **caractérisé par** les étapes de procédé suivantes :
a) fabrication, au cours d'une première étape d'un procédé de moulage par injection, d'une pièce à parois minces dotée de deux parois opposées (16) qui forme une partie intérieure (11) de la cuvette de gaz à mesurer (3),
b) introduction d'un élément de réservation, destiné à réserver un espace dépourvu de matériau injecté, dans une première et une deuxième régions des parois opposées (16) de la partie intérieure (11) qui doivent être dépourvues de matériau injecté, sachant que l'élément de réservation est orienté de telle sorte que la première région qui doit être dépourvue de matériau se trouve en alignement avec la deuxième région qui doit être dépourvue de matériau, et
c) fabrication d'une partie extérieure (10) de la cuvette de gaz à mesurer (3) au cours d'une deuxième étape du procédé de moulage par injection, par le fait que la partie intérieure (11) est entièrement enrobée de matériau par injection, sachant que deux fenêtres opposées (9), limitant le flux respiratoire, sont formées aux endroits des éléments de réservation.

2. Procédé selon la revendication 1, **caractérisé en ce que** le moule d'injection est réalisé de telle sorte que, au cours de l'étape a) du procédé, il est prévu un point d'injection pour la fabrication de la partie intérieure (11) au-dessus d'une entretoise (15), sachant que l'entretoise (15) relie les deux parois opposées (16).

3. Procédé selon la revendication 2, **caractérisé en ce que** le point d'injection pour la fabrication de la partie intérieure (11) est disposé centralement par rapport à l'entretoise (15).

4. Cuvette de gaz à mesurer (3) destinée à mesurer la concentration de composants gazeux du flux respiratoire d'un patient (1), constituée de une partie intérieure (11) avec deux parois opposées (16) qui limitent le flux respiratoire et qui laissent passer la lumière infrarouge, et
une partie extérieure (10) avec deux évidements opposés dans sa partie médiane qui forment chacun une fenêtre (9) avec les parois (16) de la partie intérieure (11), sachant que la partie extérieure (10) entoure la partie intérieure (11),
**caractérisée en ce que** la cuvette de gaz à mesurer (3) est fabriquée par un procédé selon la revendication 1.

5. Cuvette de gaz à mesurer selon la revendication 4, **caractérisée en ce que** la partie intérieure (11) et la partie extérieure (10) sont réalisées dans le même matériau.

6. Cuvette de gaz à mesurer selon la revendication 4, **caractérisée en ce que** la partie intérieure (11) et la partie extérieure (10) sont réalisées dans un matériau du groupe des polyoléfines, sachant que la partie intérieure (11) est constituée de préférence d'un copolymère aléatoire et la partie extérieure (10) de préférence d'un copolymère choc.

7. Cuvette de gaz à mesurer selon l'une des revendications 4 à 6, **caractérisée en ce qu'**une épaisseur de paroi de la partie intérieure (11) se situe dans la plage de 170 à 210 microns, et est de préférence égale à 180 microns.

8. Cuvette de gaz à mesurer selon l'une des revendications 4 à 7, **caractérisée en ce que** la partie intérieure (11) est réalisée en forme de U, de telle sorte que les parois opposées (16) sont reliées entre elles par l'intermédiaire d'une entretoise (15).

9. Cuvette de gaz à mesurer selon l'une des revendications 4 à 7, **caractérisée en ce que** la partie intérieure (11) est réalisée en forme de douille.

10. Cuvette de gaz à mesurer selon l'une des revendications 4 à 9, **caractérisée en ce que** la région médiane (7) de la partie extérieure (10) présente des moyens (12) pour la fixation d'un dispositif de mesure.

11. Cuvette de gaz à mesurer selon l'une des revendications 4 à 10, **caractérisée en ce que** la partie extérieure (10) présente un élément d'entrée (6) et un élément de sortie (8), sachant que, de préférence, des gorges s'étendant parallèlement en direction circonférentielle sont formées sur l'élément d'entrée (6).

12. Cuvette de gaz à mesurer selon la revendication 11, **caractérisée en ce que** l'élément d'entrée (6) et/ou l'élément de sortie (8) sont réalisés coniques.

13. Cuvette de gaz à mesurer selon la revendication 11 ou 12, **caractérisée en ce que** la partie intérieure (11) s'étend jusque dans l'élément d'entrée (6) et/ou l'élément de sortie (8) de la partie extérieure (10).

14. Utilisation d'une cuvette de gaz à mesurer (3) selon l'une des revendications 4 à 13 pour mesurer la concentration de composants gazeux du flux respiratoire d'un patient (1), avec un équipement de mesure qui détermine les gaz respiratoires qui s'écoulent en direction et en provenance d'un dispositif respiratoire (4) destiné à favoriser la respiration du patient (1).

15. Utilisation d'une cuvette de gaz à mesurer (3) selon la revendication 14. **caractérisée en ce que** la méthode de mesure se fonde sur une mesure de l'absorption de lumière infrarouge.
